# EUROPÄISCHE PATENTSCHRIFT

(11) **EP 0 519 293 B1**
(45) Veröffentlichungstag und Bekanntmachung des Hinweises auf die Patenterteilung: **09.12.1998**
(21) Anmeldenummer: 92109631.9
(22) Anmeldetag: 09.06.1992
(51) Int. Cl.: A61L 27/00

(54) **Implantatwerkstoff**
Implant material
Matériau d'implant

(30) Priorität: 20.06.1991 DE 4120325
(43) Veröffentlichungstag der Anmeldung: 23.12.1992
(73) Patentinhaber: MERCK PATENT GmbH, 64271 Darmstadt (DE)
(72) Erfinder: Bauer, Hans-Jörg, W-6509 Flomborn (DE); Bauer, Gerd, Dr., W-8752 Kleinostheim (DE); Dingeldein, Elvira, Dr., W-6072 Dreieich (DE); Wahlig, Helmut, Dr., W-6100 Darmstadt (DE)

(56) Entgegenhaltungen:
- EP-A- 0 061 108
- EP-A- 0 131 291
- EP-A- 0 159 036
- WO-A-88/01517
- WO-A-90/01342
- WO-A-90/01955
- DE-A- 2 620 891
- FR-A- 2 364 644
- US-A- 4 645 503
- DATABASE WPIL Week 9104, Derwent Publications Ltd., London, GB; AN 91-026448 & JP-A-2 297 374 (ASAHI OPTICAL KK)

## Beschreibung

Die Erfindung betrifft einen Implantatwerkstoff auf Basis eines Verbundmaterials von Calciumphosphatkeramik-Partikeln und bioresorbierbarem Polymer, der sich durch ein besonders günstiges Einwachs- und Einheilungsverhalten auszeichnet.

An leistungsfähiges medizinisches Implantatmaterial für den Kochenersatz wird die Forderung gestellt, daß es eine hohe mechanische Stabilität aufweist. Implantatwerkstoffe auf mineralischer Basis gewährleisten meist nur dann eine hohe mechanische Stabilität, wenn sie als Keramiken, d.h. also in Form von bei ausreichend hohen Temperaturen gesinterten Materialien bzw. Werkstücken eingesetzt werden.

Für den Einheilungsprozeß werden solche Implantatmaterialien als besonders günstig angesehen, die eine hohe Bioaktivität aufweisen, nämlich dahingehend, daß sie vom Organismus angenommen und in ihn integriert werden. Im Falle von Knochenersatzmaterial bedeutet dies, daß es bald mit körpereigenem Gewebe, insbesondere mit dem Knochen, fest und dauerhaft verwachsen soll.

Knochenersatzmaterialien ein auf Basis von Calciumphophat-Keramiken gelten aufgrund ihrer chemischen Verwandtschaft mit der Mineralphase natürlicher Knochen als bioaktiv. Natürlicher Knochen besteht in seiner Mineralphase überwiegend aus Hydroxylapatit, einem Calciumphosphat der Summenformel Ca₅(PO₄)₃OH.

Hydroxylapatit synthetischen oder organischen Ursprungs, etwa aus natürlichem Knochenmaterial, ist daher ein häufig verwendeter Rohstoff zur Herstellung von Implantaten für den Knochenersatz. Hydroxylapatit-Keramik ist im Organismus im wesentlichen nicht resorbierbar. Das heißt, das körperfremde Material bleibt über lange Zeit praktisch unverändert erhalten und die Integration in den Organismus erfolgt im wesentlichen durch Verwachsen mit vorhandenem und sich neu bildenden Knochen und Einwachsen im umgebenden Gewebe.

Die Festigkeit der Verwachsung von kompakter Calciumphosphat-Keramik mit vorhandenem Knochen ist erfahrungsgemäß überwiegend nicht befriedigend. Ein günstigeres Einwachsverhalten zeigen poröse Calciumphosphat-Keramiken.

Besonders günstig sind hierbei Materialien basierend auf natürlichem Knochen, der durch verschiedene Behandlungen mineralisiert und in ein keramisches System überführt wird, wobei die Struktur des Knochens möglichst erhalten bleiben soll. Den Verfahren gemeinsam ist die Entfernung der organischen Knochenbestandteile und die anschließende Verfestigung zur Keramik durch Sinterung bei entsprechenden Temperaturen. Die Entfernung der organischen Anteile erfolgt durch chemische Lösungsvorgänge oder durch pyrolytische Verfahren.

Knochenkeramikimplantate zeigen aufgrund ihrer ausgezeichneten Übereinstimmung mit dem Porensystem natürlichen Knochens erhebliche biologische Vorteile beim Einwachsverhalten und der Heilung in Organismus.

Formkörper aus keramischem Material, insbesondere der vorgenannten Art, werden in erster Linie für den Ersatz von tragenden Knochenstrukturen eingesetzt, die hohen mechanischen Belastungen Stand halten müssen. Beispiele hierfür sind Knochenprothesen und Knochenverbindungselemente wie etwa Markraumnägel, Knochenschrauben und Osteosyntheseplatten.

Für den Ersatz von nur wenig oder unbelasteten Knochenstrukturen wie z.B. zur Auffüllung von Spongiosa-Knochendefekten nach operativem Eingriff oder Unfall, zur Auffüllung von Zahnextraktionshöhlen oder zur plastisch-chirurgischen Behandlung von Konturdefekten im Kiefer-Gesichtsbereich, gewinnen zunehmend Verbundmaterialien auf Basis von Keramikpartikeln und physiologisch verträglichem Polymer Bedeutung. Im Vordergrund des Interesses steht bei solchen Materialien, daß sie vor oder während der Operation leicht einer Formgebung unterworfen werden können, etwa durch einfache mechanische Bearbeitung oder durch plastische Verformung.

Verbundmaterialien, hergestellt aus Keramikformkörpern und Polymer, die in den mechanischen und biologischen Eigenschaften dem natürlichen Knochen nahekommen sollen, sind ebenfalls von Interesse für die Herstellung von Implantaten mit tragenden Funktionen.

Verbundmaterialien der beschriebenen Art, insbesondere auch auf Basis von Calciumphosphatpartikeln und bioresorbierbarem Polymer, sind an sich bekannt.

In WO 90/01342 werden Implantatwerkstoffe beschrieben, die aus feinteiligen oder granulatförmigen, körperverträglichen Keramikpartikeln, insbesondere aus Hydroxylapatit anorganischen Ursprungs, in Abmischung mit körperresorbierbaren Oligomeren bzw. Polymeren niederer Hydroxycarbonsäuren, insbesondere speziell modifizierte Lactid-/Glykolid-Polymere bestehen. Je nachdem, wie diese Ausgangskomponenten ausgewählt und die Mischungsverhältnisse eingestellt werden, sind die erhaltenen Verbundmaterialien kompakt bis porös und hart bis plastisch verformbar. Als wesentliches gemeinsames Merkmal ergibt sich aber, daß das Polymer eine geschlossene Phase bildet, in der die Keramikpartikel dispergiert oder von der die Partikel zumindest vollständig umhüllt sind.

In WO 90/01955 wird ein Verbundmaterial beschrieben, das aus mineralisiertem Knochenmaterial besteht, das mit einem resorbierbaren biokompatiblen makromolekularen Material derartig imprägniert ist, daß zumindest die Oberfläche der Mineralphase bedeckt ist.

In WO-88/01517 wird eine Biokeramik-Polymer-Zusammensetzung in Pulverform, bestehend aus 70% Hydroxyapatit-Füllstoff und einem Triethylenglykoldimethacrylat-Polymer, beschrieben.

DE 3134728 beschreibt ein Material für knochenimplantate, das aus granulierter Tricalciumphosphatkeramik besteht, das mit einem Breitbandmikrobiozid imprägniert und mit einem biokompatiblen organischen Material überzogen bzw. dragiert ist.

In DE 2742128 wird ein festes, unporöses Knochenersatzmaterial beschrieben, das im wesentlichen aus einem mit einem anorganischen Füllstoff versetzten, biologisch verträglichen Polymer besteht. Als organisches Polymer werden solche auf Basis von Hydroxycarbonsäuren und als Füllstoffe Calciumphosphate offenbart. Auch in diesem Material liegt das Polymer offenbar als kontinuierliche Phase vor, in die anorganische Partikel eingebettet sind.

In den Materialien gemäß DE 2620891 sind gesinterte Calciumphosphate mit biodegradablem Polymer kombiniert. Obwohl keine näheren Angaben gemacht werden, ist davon auszugehen, daß in den beschriebenen Materialien die anorganische Komponente im wesentlichen immer von Polymer eingeschlossen, bedeckt oder überzogen ist.

Obschon die Verbundmaterialien der beschriebenen Art günstige mechanische und biologische Eigenschaften aufweisen, so sind sie doch noch verbesserungswürdig. Genauere klinische Untersuchungen haben nämlich gezeigt, daß im Einheilungsprozeß nach der Implantation im Kontaktbereich mit dem Polymermaterial zunächst bevorzugt nur Bindegewebe neu gebildet wird. Im Gegensatz hierzu stimulieren die offen vorliegenden mineralischen Kontaktflächen von Implantaten nur aus Calciumphosphatkeramik die bevorzugte Neubildung von mineralischer Knochenmatrix. Nachdem in den bekannten Verbundmaterialien die mineralische Komponente im wesentlichen immer von Polymer überzogen ist, erfolgt somit nicht, wie wünschenswert, ein direktes Auf- und Einwachsen durch neu gebildete mineralische Knochenmatrix, sondern überwiegend eine Einbettung in Bindegewebe. Hierdurch resultiert letztendlich eine nur ungenügende mechanische Festigkeit des Übergangs von Implantat zu körpereigener Knochensubstanz.

Aufgabe der Erfindung war es nun, einen verbesserten Verbundwerkstoff für den Knochenersatz aufzufinden, der bei zumindest gleichermaßen guten Gesamteigenschaften in bezug auf mechanische Festigkeit, Bearbeitbarkeit und Bioaktivität ein besonders günstiges Einwachs- und Einheilungsverhalten zeigt.

Es wurde nun gefunden, daß dies erreicht wird mit einem Implantatwerkstoff auf Basis eines Verbundmaterials von Calciumphosphatkeramik-Partikeln und bioresorbierbarem Polymer, in dem der Anteil von Calciumphosphatkeramik-Partikeln mindestens 50 Gew.% beträgt und die Partikel miteinander durch Polymerstege zu einer dreidimensionalen, offenporigen Struktur verbunden sind, worin die Partikeloberflächen zu höchstens 50 % mit Polymer bedeckt sind erhältlich durch Erhitzen eines Gemisches der Komponenten mittels Mikrowellenstrahlung. Es hat sich gezeigt, daß in dem erfindungsgemäßen Verbundwerkstoff aufgrund des Vorliegens von Calciumphosphatkeramik-Partikeln mit einem hohen Anteil an freier, nicht mit Polymer bedeckter Oberfläche im Verbund mit der durch Polymerstege gebildeten dreidimensionalen, offenporigen Struktur in unerwartet hohem Maße die Neubildung von mineralischer Knochenmatrix und damit eine besonders feste und innige Verwachsung mit der körpereigenen Knochensubstanz erfolgt.

Der erfindungsgemäße Implantatwerkstoff läßt sich in einfacher Weise herstellen durch Erhitzen eines Gemisches der Komponenten mittels Mikrowellenstrahlung. Hierbei bildet das erschmelzende und danach wieder erstarrende Polymer mit den Calciumphosphatkeramik-Partikeln eine dreidimensionale, offenporige Struktur, in der die Keramikpartikel miteinander durch Polymerstege verbunden sind. Bei einem Anteil an Keramikpartikeln von mindestens 50 Gew.% und dem sich ausbildenden Verbund über Polymerstege wird gewährleistet, daß die Partikeloberflächen zu höchstens 50 % mit Polymer bedeckt sind. Eine für die praktische Anwendung sehr günstige Eigenschaft des erfindungsgemäßen Implantatwerkstoffes ist, daß er sich nach erneutem Erhitzen mit Mikrowellenstrahlung plastisch verformen läßt, ohne daß sich hierbei die Struktur des Materials verändert.

Gegenstand der Erfindung ist somit ein Implantatwerkstoff auf Basis eines Verbundmaterials von Calciumphosphatkeramik-Partikeln und bioresorbierbarem Polymer, der dadurch gekennzeichnet ist, daß in dem Werkstoff der Anteil von Calciumphosphatkeramik-Partikeln mindestens 50 Gew.% beträgt und die Partikel miteinander durch Polymerstege zu einer dreidimensionalen, offenporigen Struktur verbunden sind, worin die Partikeloberflächen zu höchstens 50 % mit Polymer bedeckt sind, erhältlich durch Erhitzen eines Gemisches der Komponenten mittels Mikrowellenstrahlung.

Gegenstand der Erfindung ist schließlich ein Verfahren zur Formgebung eines derartigen Implantatwerkstoffes, wobei man diesen mittels Mikrowellenstrahlung bis zur plastischen Verformbarkeit erhitzt und dann mechanisch verformt.

Als Ausgangskomponenten für den erfindungsgemäßen Implantatwerkstoff können grundsätzlich alle auf dem einschlägigen Gebiet bekannten Calciumphosphatkeramik-Materialien und bioresorbierbaren Polymermaterialien eingesetzt werden.

Als entsprechende Keramikmaterialien sind im wesentlichen solche zu verstehen, in denen das zugrundeliegende Calciumphosphat ein Ca:P-Verhältnis zwischen 1,0 und 2,0 aufweist und die bei einer ausreichend hohen Temperatur, in der Regel im Bereich zwischen ca. 800 und 1500 °C, zur Keramik gesintert worden sind. Die zugrundeliegenden Calciumphosphate können synthetischen Ursprungs, beispielsweise aus der Umsetzung von Calciumoxid mit Phosphorsäure im entsprechenden molaren Verhältnis, oder natürlichen Ursprungs, beispielsweise aus mineralischer oder organischer Quelle sein. Typische Calciumphosphate sind Hydroxylapatit, Tricalciumphosphat und Tetracalciumphosphat sowie deren Um- und Abwandlungsprodukte, die in der Keramik auch als Mischphasen nebeneinander vorliegen können. Bevorzugt sind Calciumphosphate organischen Ursprungs, insbesondere Hydroxylapatit aus natürlichem Knochen. Letzteres kann aus Knochen durch Mineralisation nach an sich bekannten Methoden gewonnen werden.

Die Calciumphosphatkeramik-Partikel für das erfindungsgemäße Verbundmaterial können in Pulver- bis Granulatform eingesetzt werden, wobei die Partikelgröße beliebig zwischen 20 µm und 5 mm gewählt werden kann. Besonders bevorzugt sind Granulate mit Partikelgrößen zwischen 0,1 und 3 mm, insbesondere zwischen 0,5 und 1,5 mm.

Die Partikel können kompakt, also mit geringer Porosität, oder porös sein. Ersteres trifft vornehmlich bei pulvrigen Materialien zu, letzteres wird vorzugsweise bei Granulaten realisiert.

Bei porösen Materialien kann eine Porosität bis 90 % des Partikelvolumens vorliegen, wobei eine offene Porosität bevorzugt ist. Die Materialien können, je nach Herkunft und Partikelgröße, eine Mikroporosität mit Porengrößen zwischen 1-100 µm und/oder eine Makroporosität mit Porengrößen bis etwa 3 mm aufweisen.

Aufgrund der besonderen Übereinstimmung mit natürlichem Knochen hinsichtlich chemischer Zusammensetzung, Kristallit- und Porenstruktur sind Knochenkeramikpartikel besonders günstig als Keramikkomponente in dem erfindungsgemäßen Verbundmaterial. Typischerweise kommen die Knochenkeramikpartikel in Form von hochporösem Spongiosa-Granulat oder in Form von Corticalisgranulat, das eine geringe Porosität aufweist, zur Anwendung. Dem Fachmann sind diese Materialien, ihre Herstellung, Modifizierung, Verarbeitung und Anwendung geläufig bzw. problemlos aus der einschlägigen Fachliteratur, wie beispielsweise den eingangs zitierten Schriften, entnehmbar.

Als bioresorbierbare Polymere kommen synthetische Polymere und natürliche, hochmolekulare Materialien, die auch in bekannter Weise chemisch modifiziert sein können, in Betracht. Typische natürliche, hochmolekulare Stoffe sind Polysaccharide, wie Stärke, Cellulose und Derivate hiervon, Proteine, wie Gelatine und Collagen, oder Triglyceride höherer Alkancarbonsäuren, wie hochschmelzende Fette und Wachse. Typische synthetische Polymere sind vornehmlich Oligomere und polymere Ester von Hydroxycarbonsäuren, wie insbesondere von Milchsäure und Glycolsäure. Dem Fachmann sind auch diese Materialien, ihre Herstellung, Modifizierung, Verarbeitung und Anwendung geläufig bzw. problemlos aus der einschlägigen Fachliteratur entnehmbar. Als Hinweis können wiederum die eingangs zitierten Schriften und die darin angegebene Literatur dienen. Besonders bevorzugt als Polymerkomponente in dem erfindungsgemäßen Verbundmaterial sind Polymermaterialien auf Basis von Polylactiden und/oder Polyglycoliden. Diese Materialien können Homopolymere von D-, L- und D,L-Milchsäure sowie von Glycolsäure, Copolymere oder Gemische hiervon und Gemische mit entsprechenden Oligomeren und Monomeren darstellen. Je nach Wahl der chemischen Zusammensetzung, des Polymerisationsgrades, des Anteils an Oligomeren und Monomeren oder sonstiger üblicher Modifizierungen und Zusätze kann die Konsistenz des Polymermaterials zwischen spröde-hart, weich-elastisch und zäh-viskos eingestellt werden. Zweckmäßigerweise liegt die mittlere Molmasse des Polymermaterials zwischen etwa 200 und 10.000 und vorzugsweise zwischen etwa 1.000 und 3.000. Zweckmäßigerweise sollte das Polymermaterial in einem Temperaturbereich schmelzbar sein, der nicht über 180 °C hinausreicht. Idealerweise wird das Polymer so ausgewählt oder eingestellt, daß es bei Raumtemperatur von im wesentlichen harter Konsistenz ist aber bei Temperaturen zwischen etwa 40 und 60 °C soweit erweicht, daß es plastisch verformbar wird.

Entscheidendes Merkmal des erfindungsgemäßen Verbundmaterials ist die dreidimensional offenporige Struktur, die so ausgebildet ist, daß die Calciumphosphatkeramik-Partikel durch Polymerstege miteinander verbunden sind, wobei die Partikeloberflächen zu höchstens 50 % mit Polymer bedeckt sind.

Die Porengröße des Verbundmaterials kann bis zu etwa 3 mm betragen und liegt vorzugsweise im Bereich von 0,01-1 mm.

Wesentliche Voraussetzung für die Ausbildung einer derartigen Struktur ist, daß in dem Verbundwerkstoff der Anteil an Calciumphosphatkeramik-Partikeln mindestens 50 Gew.% beträgt. Vorzugsweise liegt der Anteil an Calciumphosphatkeramik-Partikeln zwischen 75 und 95 Gew.%. Als obere Grenze für ein Material mit noch brauchbarem Zusammenhalt sind 98 Gew.% anzusetzen. Das Verhältnis von Keramikpartikeln zu Polymer kann, in Abhängigkeit von der Natur der Komponenten, insbesondere von der Porosität der Partikel, optimiert werden. So ist es besonders günstig, im Falle von hochporösem Spongiosa-Keramikgranulat nicht mehr als höchstens 80 Gew.% vorzusehen, da ein gewisser Anteil an Polymer von dem porösen Material aufgenommen wird. Im Falle von Corticalis-Keramikpartikeln mit geringer Porosität kann der Anteil höher gewählt werden, höchstens jedoch 90 Gew.%.

Eine weitere wesentliche Voraussetzung für die Ausbildung der Struktur ist die Art der Herstellung des Verbundmaterials durch Erhitzen eines entsprechenden Gemisches der Komponenten mittels Mikrowellenenergie. Hierbei wird ein rasches Aufschmelzen des Polymermaterials bewirkt, bei dem sich unter den eigenen Kohäsionskräften viele einzelne separierte und nicht zusammenfließende tropfenartige Polymerdomänen bilden, die die Keramikpartikel zu einem dreidimensionalen Netzwerk mit stegartiger Verknüpfung verbinden. Es wird angenommen, daß diese Ausbildung der stegartigen Verknüpfung durch eine selektive und schlagartige, im gesamten Volumen gleichzeitig wirkende Erhitzung des Polymermaterials durch die Mikrowellenenergie bewirkt wird, während sich die Keramikpartikel im wesentlichen nicht aufheizen. Aus diesem Grund kann die Erhitzungsdauer auch relativ kurz gewählt werden. Wegen des durchdringenden Charakters der Mikrowellenenergie ist die Erhitzungsdauer auch weitgehend unabhängig von Menge und Volumen des Materialgemisches, allenfalls von der Mikrowellenleistung. Die einzustrahlende Mikrowellenleistung ist zweckmäßigerweise in erster Linie auf den Schmelztemperaturbereich des Polymers abzustellen. Als Quellen für die Mikrowellenenergie kommen Mikrowellenöfen, wie sie für den Haushaltsbereich gängig sind, in Frage. Diese arbeiten üblicherweise bei einer Frequenz von 2,45 GHz und mit Leistungen zwischen 450 und 1000 Watt. Die Behandlungsdauer des Materials mit solchen Geräten liegt je nach eingestellter Mikrowellenleistung im Minutenbereich, typisch zwischen 2 und 10 Minuten. Die entsprechenden Arbeitsbedingungen lassen sich ansonsten ohne weiteres durch einfache Routineversuche ermitteln und für den Einzelfall optimieren.

Für die praktische Herstellung des erfindungsgemäßen Verbundmaterials werden die Komponenten Calciumphosphatkeramik-Partikel und Polymermaterial zunächst im gewünschten Verhältnis innig gemischt. Je nach Natur kann das Polymermaterial als Pulver, Granulat oder plastisch-pastöse Masse vorliegen. Die Durchmischung erfolgt jeweils mit den Materialien angepaßten Methoden und Geräten. Das Gemisch wird dann zweckmäßigerweise in ein formgebendes Gefäß verbracht, das aus einem inerten, mikrowellenresistenten Material besteht. Die Formgebung kann auf den späteren Einsatz des Implantats abgestellt sein. Es können aber auch lediglich Rohlinge geformt werden, aus denen später dann für den jeweiligen Einsatzzweck entsprechende Implantatformkörper gearbeitet werden. Durch Behandlung mit Mikrowellenenergie in einem handelsüblichen Gerät bei z.B. 450 Watt und einer Dauer von 3-5 Minuten bildet sich der erfindungsgemäße Verbundwerkstoff mit der vorbeschriebenen Struktur heraus. Der abgekühlte Verbundwerkstoff ist strukturstabil und kann problemlos gelagert werden.

Zwar ist es in einzelnen Fällen auch möglich, durch empirische Auswahl von Ausgangskomponenten und Mischungsverhältnissen Zusammensetzungen einzustellen, die sich auch durch Zufuhr der erforderlichen Wärmeenergie mit anderen Heizquellen, wie z.B. konventionellen Öfen oder IR-Strahlern, zu einem Verbundmaterial vergleichbarer Struktur verarbeiten lassen. Die Steuerung der Wärmezufuhr ist hierbei insgesamt jedoch äußerst kritisch und in der Regel kommt es zu einem vollständigen Zusammenschmelzen des Polymermaterials, was zu einem weitgehenden bis vollständigen Überzug bzw. Einschluß der Keramikpartikel durch das Polymer führt. Als sichere, über den gesamten Zusammensetzungsbereich und alle Varianten der Ausgangsmaterialien erstreckende Methode hat sich erfindungsgemäß jedoch nur die Erhitzung mittels Mikrowelle erwiesen.

Je nach eingestellter Konsistenz kann das Material durch unterschiedliche Maßnahmen formgebend bearbeitet werden. Werkstoffe mit vorwiegend fester Konsistenz können durch mechanische Bearbeitung wie Sägen, Schneiden, Feilen, Fräsen mit hierfür üblichen Werkzeugen bearbeitet werden. Materialien, die mehr plastisch-weich eingestellt sind, lassen sich auch durch mechanischen Druck verformen. Besonders zweckmäßig sind Werkstoffe, die so eingestellt sind, daß sie bei Raum- bzw. Körpertemperatur fest sind und durch leichtes Erhitzen bis zur plastischen Verformbarkeit erweichen. Besonders günstig ist für diesen Erhitzungsschritt wiederum der Einsatz von Mikrowellenenergie. Hierdurch wird analog zum Herstellungsprozeß sicher gewährleistet, daß die dreidimensionale, offenporige, durch Polymerstege gebildete Netzwerkstruktur des Materials und die weitgehende Freiheit der Keramikpartikeloberflächen von Polymer erhalten bleibt. Derartige durch Mikrowellenenergie plastifizierbare Verbundwerkstoffe lassen sich durch mechanischen Druck verformen und so gut den lokalen Gegebenheiten am Implantationsort anpassen, etwa bei einer Auffüllung von Spongiosa-Knochendefekten, Zystenhöhlen, Zahnextraktionshöhlen sowie bei der Verbindung oder beim Ersatz von Knochenbruchstücken. Die Verformbarkeit bleibt in der Regel für einen verarbeitungsgerechten Zeitraum, der im Minutenbereich liegt, erhalten. Nach dem Erkalten hat das Material wieder seine ursprüngliche feste Konsistenz.

Das erfindungsgemäße Verbundmaterial ist vorzüglich geeignet als Implantatwerkstoff für den Knochenersatz, da er sich, wie sich in klinischen Modellversuchen zeigt, durch ein besonders günstiges Einwachs- und Einheilungsverfahren auszeichnet. Dies liegt offenbar an den einzigartigen Strukturmerkmalen, die von der Zusammensetzung her vergleichbare Materialien nach dem Stand der Technik nicht aufweisen.

Die dreidimensionale offenporige Struktur, die in besonderem Maße der Struktur natürlicher spongiöser Knochensubstanz entspricht, fördert das Einsprossen von sich neubildendem Knochengewebe, wobei sich ein inniger Verbund mit dem Implantat ausbildet.

Die Vorteile von bioresorbierbaren Polymermaterialien und von Calciumphosphatkeramik in Implantaten sind an sich allgemein anerkannt. Der hohe Anteil von freiliegender Oberfläche der Calciumphosphatkeramik-Partikel von mindestens 50 % und vorzugsweise 75-95 % fördert die bevorzugte Neubildung und das Aufwachsen von körpereigener mineralischer Knochensubstanz bei reduzierter Bindegewebsneubildung.

Die Anwendung des erfindungsgemäßen Verbundmaterials als Implantatwerkstoff für den Knochenersatz ist vielfältig. Je nach Festigkeit und mechanischer Belastbarkeit des Werkstoffes können daraus vorgefertigte Implantatformkörper, etwa für den Ersatz von definierten Knochenteilen, hergestellt oder das Material kann zur Auffüllung von Knochenhöhlen und für die Rekonstituierung von nichttragenden Knochenbereichen verwendet werden.

Das letztere Anwendungsgebiet ist ein bevorzugter Einsatzbereich des erfindungsgemäßen Verbundmaterials. Im Vordergrund steht hierbei die Auffüllung von Zahnextraktionshöhlen, die Rekonstituierung von Konturdefekten im Kiefer- und Gesichtsbereich sowie die Auffüllung von Spongiosa-Knochendefekten nach krankheitsbedingtem operativem Eingriff oder bei der Unfallchirurgie.

In die erfindungsgemäßen Verbundmaterialien können auch pharmazeutische Wirkstoffe, wie sie für entsprechende Anwendungen bekannt und üblich sind, inkorporiert werden. Zweckmäßig sind etwa antibiotisch und/oder cytostatisch wirkende Pharmazeutika zur Unterdrückung bzw. Bekämpfung von Infektionen oder für die Krebstherapie. Weiterhin können Wirkstoffe zum Einsatz gelangen, die den Einheilungsprozeß fördern, wie etwa zell-, gefäß- und knochenwachstumsfördernde Mittel. Geeignet sind etwa entspreched wirksame Peptid-Wachstumsfaktoren, Vitamine und Hormone. Es ist zweckmäßig, den einzusetzenden Wirkstoff oder die Wirkstoffkombination in der zweckentsprechenden Dosierung zunächst in das Polymermaterial einzuarbeiten und dann daraus das erfindungsgemäße Verbundmaterial zu fertigen.

### Beispiel

75 Gew.-Teile eines Granulats aus Spongiosa-Knochenkeramik mit einer Korngröße von 0,5-1,25 mm werden nacheinander mit 22 Gew.-Teilen Poly-(D,L)-Lactid mit einer Molmasse von 2000 und mit 3 Gew.-Teilen (D,L)-Milchsäure innig vermischt. Die Masse wird in eine mit Teflon beschichtete Form gegeben und mit leichtem Druck auf die Oberfläche verdichtet. Die gefüllte Form wird in ein handelsübliches Mikrowellengerät mit Drehteller außermittig gesetzt und 3,5 Minuten mit einer Energie von 450 Watt behandelt. Nach Entnahme läßt man unter Feuchtigkeitsabschluß abkühlen. Man erhält einen festen, offenporigen Implantatwerkstoff, bei dem die Keramikgranulatkörner durch Polymerstege verbunden sind. Etwa 80 % der Granulatoberfläche ist von Polymer unbedeckt.

Durch erneute Mikrowellenbehandlung wird das Material für einen Zeitraum von etwa 3 Minuten plastisch verformbar und verfestigt sich dann wieder.

## Patentansprüche

1. Implantatwerkstoff auf Basis eines Verbundmaterials von Calciumphosphatkeramik-Partikeln und bioresorbierbarem Polymer, in dem der Anteil von Calciumphosphatkeramik-Partikeln mindestens 50 Gew.% beträgt und die Partikel miteinander durch Polymerstege zu einer dreidimensionalen offenporigen Struktur verbunden sind, worin die Partikeloberflächen zu höchstens 50 % mit Polymer bedeckt sind, erhältlich durch Erhitzen eines Gemisches der Komponenten mittels Mikrowellenstrahlung.

2. Implantatwerkstoff nach Anspruch 1, dadurch gekennzeichnet, daß er aus gesinterten Knochenkeramik-Partikeln und aus Polymermaterial auf Basis von Polylactiden und/oder Polyglycoliden besteht.

3. Implantatwerkstoff nach Anspruch 2, dadurch gekennzeichnet, daß die enthaltenden Knochenkeramik-Partikel porös, vorzugsweise mit offener Porosität, sind.

4. Implantatwerkstoff nach Anspruch 3, dadurch gekennzeichnet, daß er als poröse Knochenkeramik-Partikel gesintertes Spongiosa-Granulat enthält.

5. Implantatwerkstoff nach den Ansprüchen 1 bis 4, dadurch gekennzeichnet, daß der Anteil von freiliegender Oberfläche der Calciumphosphatkeramik-Partikel 75-95 % beträgt.

6. Verfahren zur Herstellung eines Implantatwerkstoffes auf Basis eines Verbundmaterials aus Calciumphosphatkeramik-Partikeln und bioresorbierbarem Polymer, in dem die Partikel miteinander durch Polymerstege zu einer dreidimensional offenporigen Struktur verbunden sind, worin die Partikeloberflächen zu höchstens 50 % mit Polymer bedeckt sind, durch Erhitzen eines Gemisches der Komponenten, in dem der Anteil an Calciumphosphatkeramik-Partikeln mindestens 50 Gew.% beträgt, mittels Mikrowellenstrahlung.

7. Verfahren nach Anspruch 6, dadurch gekennzeichnet, daß man ein Gemisch aus gesinterten Knochenkeramik-Partikeln mit einem Polymermaterial auf Basis von Polylactiden und/oder Polyglycoliden einer Mikrowellenstrahlung aussetzt.

8. Verfahren zur Formgebung eines Implantatwerkstoffes gemäß den Ansprüchen 1 bis 5, wobei man diesen mittels Mikrowellenstrahlung bis zur plastischen Verformbarkeit erhitzt und dann durch mechanischen Druck verformt.

## Claims

1. Implant material based on a composite material of calcium phosphate ceramic particles and bioabsorbable polymer, in which the proportion of calcium phosphate ceramic particles is at least 50% by weight and the particles are joined to one another by polymer bridges to give a three-dimensional open-pore structure in which the particle surfaces are covered with polymer to the extent of not more than 50%, obtainable by heating a mixture of the components by means of microwave radiation.

2. Implant material according to Claim 1, characterised in that it consists of sintered bone ceramic particles and of polymer material based on polylactides and/or polyglycolides.

3. Implant material according to Claim 2, characterised in that the bone ceramic particles contained are porous, preferably having open porosity.

4. Implant material according to Claim 3, characterised in that it contains sintered spongiosa granules as the porous bone ceramic particles.

5. Implant material according to Claims 1 to 4, characterised in that the proportion of exposed surface of the calcium phosphate ceramic particles is 75-95%.

6. Process for the preparation of an implant material based on a composite material of calcium phosphate ceramic particles and bioabsorbable polymer, in which the particles are joined to one another by polymer bridges to give a three-dimensionally open-pore structure in which the particle surfaces are covered with polymer to the extent of not more than 50%, by heating a mixture of the components, in which the proportion of calcium phosphate ceramic particles is at least 50% by weight, by means of microwave radiation.

7. Process according to Claim 6, characterised in that a mixture of sintered bone ceramic particles with a polymer material based on polylactides and/or polyglycolides is exposed to microwave radiation.

8. Process for shaping an implant material according to Claims 1 to 5, in which this is heated to plastic deformability by means of microwave radiation and is then deformed by mechanical pressure.

## Revendications

1. Matériau d'implant à base d'un matériau composite de particules de céramique de phosphate de calcium et de polymère biorésorbable, dans lequel la proportion de particules de céramique de phosphate de calcium s'élève à au moins 50% en poids et les particules sont reliées entre elles par des traverses polymères à une structure tridimensionnelle à pores ouverts, où la surface des particules est recouverte à au plus 50% par un polymère, que l'on peut obtenir par chauffage d'un mélange des composants au moyen d'une irradiation par micro-ondes.

2. Matériau d'implant selon la revendication 1, caractérisé en ce qu'il se compose de particules de céramique osseuse frittée et de matériau polymère à base de polylactides et/ou de polyglycolides.

3. Matériau d'implant selon la revendication 2, caractérise en ce que les particules de céramique osseuses contenues sont poreuses, de préférence à porosité ouverte.

4. Matériau d'implant selon la revendication 3, carcatérisé en ce qu'il contient comme particules de céramique osseuse poreuse un granulé de matière spongieuse fritté.

5. Matériau d'implant selon les revendications 1 à 4, caractérisé en ce que la proportion de surface exposée des particules de céramique de phosphate de calcium s'élève à 75-95%.

6. Procédé de fabrication d'un matériau d'implant à base d'un matériau composite fait de particules de céramique de phosphate de calcium et de polymère biorésorbable, dans lequel les particules sont reliées entre elles par des traverses polymères en une structure tridimensionnelle à pores ouverts, la surface des particules étant recouverte à au plus 50% avec un polymère, par chauffage d'un mélange des composants, dans lequel la proportion des particules de céramique de phosphate de calcium s'élève à au moins 50% en poids, au moyen d'une irradiation par micro-ondes.

7. Procédé selon la revendication 6, caractérise en ce qu'on expose à une irradiation par micro-ondes un mélange de particules de céramique osseuse fritté avec un matériau polymère à base de polylactides et/ou de polyglycolides.

8. Procédé de formage d'un matériau d'implant selon les revendications 1 à 5, dans lequel on chauffe ce matériau au moyen d'une irradiation par micro-ondes jusqu'à lui donner une déformabilité plastique, puis on le déforme par pression mécanique.
